(19)

Europäisches Patentamt

European Patent Office

Office européen des brevets

(11) **EP 4 647 418 A1**

(12) **EUROPEAN PATENT APPLICATION**

(43) Date of publication:
**12.11.2025 Bulletin 2025/46**

(21) Application number: **24382504.9**

(22) Date of filing: **08.05.2024**

(51) International Patent Classification (IPC):
*C07C 1/04* (2006.01)    *C07C 15/06* (2006.01)
*C07C 15/04* (2006.01)    *C07C 15/08* (2006.01)
*B01J 23/78* (2006.01)    *B01J 29/40* (2006.01)

(52) Cooperative Patent Classification (CPC):
(C-Sets available)
**C07C 1/044; B01J 23/78; B01J 29/40;**
**C07C 1/0445;** C07C 2523/04; C07C 2523/72;
C07C 2523/745; C07C 2523/78; C07C 2529/40
(Cont.)

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Universidad de Sevilla**
**41013 Sevilla (ES)**

(72) Inventors:
• **BLAY ROGER, José Rubén**
**41013 Sevilla (ES)**

• **NAWAZ, Asif**
**41013 Sevilla (ES)**
• **RAMÍREZ REINA, Tomás**
**41013 Sevilla (ES)**
• **BOBADILLA BALADRÓN, Luis**
**41013 Sevilla (ES)**
• **ODRIOZOLA GORDÓN, José Antonio**
**41013 Sevilla (ES)**

(74) Representative: **Herrero & Asociados, S.L.**
**Edificio Aqua - Agustín de Foxá, 4-10**
**28036 Madrid (ES)**

(54) **ONE POT PROCESS FOR SYNTHESIZING AROMATIC HYDROCARBONS FROM SYNGAS**

(57) The present invention relates to an improved process of efficiently synthesizing value-added chemicals, i.e. aromatic hydrocarbons, from syngas in one pot process by metal-based/zeolite composite catalyst. The invention especially relates the manufacturing of lower aromatic hydrocarbons such as benzene, toluene, xylenes (BTX) from syngas fractions ($CO/CO_2/H_2$) via catalytic treatment.

**FIG. 1**

(52) Cooperative Patent Classification (CPC): (Cont.)

C-Sets
**C07C 1/044, C07C 15/04;**
**C07C 1/044, C07C 15/06;**
**C07C 1/044, C07C 15/08;**
**C07C 1/0445, C07C 15/04;**
**C07C 1/0445, C07C 15/06;**
**C07C 1/0445, C07C 15/08**

**Description**

**FIELD OF THE INVENTION**

The present invention relates to an improved process of efficiently synthesizing value-added chemicals, i.e. aromatic hydrocarbons, from gas that comprises $H_2$ and CO in one pot process by metal-based/zeolite composite catalyst. The invention especially relates the manufacturing of lower aromatic hydrocarbons such as benzene, toluene, xylenes (BTX).

**BACKGROUND OF THE INVENTION**

Aromatic hydrocarbons especially BTX (benzene, toluene, xylene), being conventionally obtained as a by-product of gasoline or ethylene production units through distillate fractions in the petroleum industries, are extensively used as the essential chemical raw materials in aerospace, defense, and energy vector. At present, the major production routes of aromatics divert towards the catalytic reforming of naphtha or steam cracking, hydrolysis, and catalytic cracking of light cycle oil, etc., while adopting the petroleum resources as raw material.

Other source to obtain aromatic hydrocarbon has been natural gas. The patent US10550045 is relates to a method for producing aromatic hydrocarbons from natural gas, which involve producing synthesis gas, converting the same into methanol, producing, from the methanol, in the presence of a catalyst, a concentrate of aromatic hydrocarbons and water, separating the water, air stripping hydrocarbon residues from the water, and separating-out the resultant concentrate of aromatic hydrocarbons and hydrogen-containing gas, the latter being at least partially used in the production of synthesis gas to adjust the ratio therein of $H_2$:CO 1.8-2.3:1. The disclosed process comprises multiple steps and exhibits low selectivity in the production of aromatic hydrocarbons.

The syngas is also a source to obtain aromatic hydrocarbons. The Canadian patent CA1175073A discloses a process for preparing a hydrocarbon mixture, rich in aromatic hydrocarbons. The syngas is subjected to a catalytic reaction with a mixed catalyst comprising a physical mixture of (1) a palladium catalyst comprising palladium supported on a carrier and (2) a zeolite catalyst, thereby to produce an aromatics-rich hydrocarbon mixture. The method yields a mixture of hydrocarbon wherein the aromatics are lower than 60%.

The production efficiency of these aromatic hydrocarbons is not high enough. Even though, employing different processes of Aroforming, M2 forming process; Z-forming process, Cyclar process of LPG (Liquefied Petroleum Gas) or $C_3$-$C_4$ naphtha unit, AROMAX process or RZ-Platformer process etc., could produce aromatics as targeted products, however, still lacking far behind in the yield's static.

The aromatics production technology directly from biomass or coal derived syngas being regarded as syngas to aromatics (STA), could help in compensating the global consumption of those petroleum derivatives, while alleviating the demand gap of high-value aromatics and the production cost. Since various catalytic processes of utilizing alternative resources for producing value added energy-related products and chemicals have been widely discovered, where the exemplary method of gas to liquid (GtL) through Fischer-Tropsch synthesis (FTS) or methanol synthesis (MTS) processes are highly desirable in the renewable anergy regimes. However, the kinetics control by Anderson-Schulz-Flory (ASF) rule, and extensive number of byproducts ($CO_2$, etc), need to be optimized for improving the overall efficiency of existing technologies.

The effective advancement and appealing strategies in the decades back presented route of STA process by adopting the variety of multi-functional catalyst scheme in a single stage, have significantly invaded the activity barriers (in terms of performance, thermodynamics, and cost etc.) that usually encounter in conventional twostep process. However, limited to the laboratory exploration stage of one-step STA unit, there are still relatively few studies in this era that need the exploration of appropriate solutions for the development of high conversion, stability, and targeted aromatics selective catalytic system. There remains a wide gap of unravelling a rationally designed hybrid catalyst with high activity and high stability to directly transform syngas to highly selective (over 90%) formation of desired aromatic hydrocarbons in a single-stage (one-pot) process.

Various investigations disclosed different methods of improving the catalyst life and aromatics production with a mixture of CO, $CO_2$, and $H_2$ reaction gas, however, failing to achieve considerable yield. In most cases, if the catalyst life could sustain longer, the yield of the aromatic hydrocarbon is suppressed to be low level especially at the industrial scale. Some studies have disclosed different methods of catalytic evolution of excellent physiochemical behaviours and oxygen-vacancy-rich spinel structured integrated with HZSM-5 molecular sieves to achieve one-step STA process, where the aromatics selectivity can reach (50%-85%), however either have encountered the low CO conversion levels of 20-40%) or finding relatively high $CO_2$ generations (30-50%).

While other reaching to the high conversion rate (50%), with more than 80% aromatics selectivity in one-step aromatic synthesis schemes, however, mainly diverging towards higher molecules fractions polymethyl benzene or durene, with reduced light aromatics (BTX) selectivity. Various documents adopting iron-based metal oxide catalysts, find the good initial conversion and aromatics selectivity, but relatively poor stability with sharply dropped catalyst performance with the extension of the reaction time, making a certain distance from the requirements of industrial applications. Therefore, necessary examinations on the catalyst life are direly needed to be carried out for the development of different advanced techniques that should be capable of keeping a high yield of the aromatic hydrocarbon for a long period of time.

## SUMMARY OF THE INVENTION

The process referred in the present invention offers a distinct advantage in maintaining a consistently high yield of aromatic hydrocarbons over prolonged durations combined with a high rate of CO conversion level, owing to the sustained efficiency of the catalyst employed throughout the extended operational period.

In the present invention has been development a process to obtain aromatic from a syngas result in a high CO conversion rate of over 96% and while efficiently reaching the aromatics content to 98.72%, with the major fraction of benzene, toluene and xylene (BTX). Another advantage of the present process is that the catalyst maintains the activity for a long period of time. The process is one pot process, this term refers to a multi-step chemical reaction carried out in a reaction vessel.

The composite catalyst used in the process of the invention, that comprises an alkali-Fe- transition metal oxide and a nano molecular sieve zeolite, realizes the enhanced conversion efficiency of the gas and reduces side reactions by effective improvement in the intermediate products. The alkali increases the activity of the catalyst. The bimetallic Fe-transition metal based solid oxide along with the alkali afford the excellent stability and selectivity of the low carbon olefins in the intermediate product that are beneficial in generating the targeted aromatic hydrocarbons.

In the present process the highly selective aromatic synthesis is obtained through the optimization of various reaction parameter.

Therefore, the first aspect of the present invention refers to a process to obtain aromatic hydrocarbons, from a syngas, comprising the steps of:

a) preheating a composite catalyst to a temperature lower than a reaction temperature;
b) reducing the composite material;
c) reacting in a fixed bed the gas at the reaction temperature from 150°C to 350°C, at a pressure from $1 \cdot 10^5$ Pa to $5 \cdot 10^6$ Pa and at a gas hour space velocity (GHSV) is in a range from 1000 mLg$^{-1}$h$^{-1}$ to 5000 mLg$^{-1}$h$^{-1}$ in the presence of the composite catalyst;

wherein the composite catalyst comprising:

an alkali-Fe- transition metal oxide wherein the Fe is from 50% to 90% by weight relative to the total weight of the alkali-Fe- transition metal oxide and
a nano molecular sieve zeolite;
and wherein the mass ratio of the metal oxide to the nanosized molecular sieve zeolite is (0.1-10):1.

The term syngas a "syngas" or "synthesis gas" means synthesis gas which is the name given to a gas mixture that contains varying amounts of carbon monoxide, hydrogen and/ or carbon dioxide. The raw syngas containing $CO_2$ and the composition is for example 20.0% $H_2$, 10.0% CO, 5.0% $CO_2$, and balance $N_2$, and the pure syngas does not contain $CO_2$ and the composition is for example 20.0% $H_2$, 10.0% CO and balance $N_2$.

The term "aromatic hydrocarbon" is very well known in the art. Accordingly, the term "aromatic hydrocarbon" relates to cyclically conjugated hydrocarbon, containing at least one benzene moiety; such benzene moieties may be substituted or unsubstituted. Examples of aromatic hydrocarbon are benzene, xylene and toluene.

The term "molecular sieve zeolite" refers to a crystalline aluminosilicate material with a highly ordered structure, characterized by regularly spaced pores of molecular dimensions.

## BRIEF DESCRIPTION OF THE DRAWINGS

For the purpose of aiding the understanding of the characteristics of the invention, according to a preferred practical embodiment thereof and in order to complement this description, the following figures are attached as an integral part thereof, having an illustrative and non-limiting character.

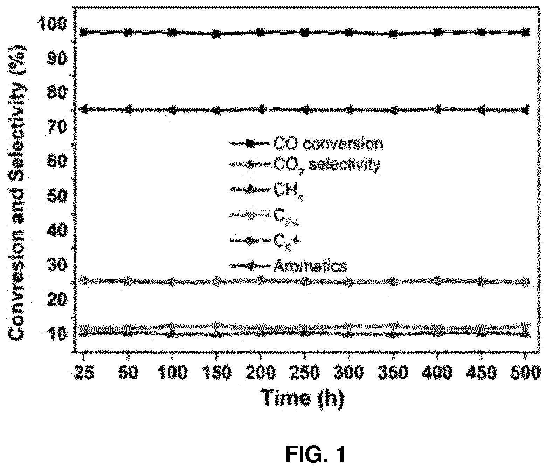

The Figure 1 includes a chart showing the effect of time on stream (TOS) on CO conversion, light hydrocarbon selectivity, $CO_2$ selectivity and liquid product selectivity for an Na modified Fe-Cu based composite catalyst with HZSM-5 zeolite at the reaction conditions of 300° C, 30 bar, and 2000 h$^{-1}$ with syngas (20.0% $H_2$, 10.0% CO, 5.0% $CO_2$, and balance $N_2$).

## DETAILED DESCRIPTION OF THE INVENTION

As it is said above the first aspect of the present invention refers to process to obtain aromatic hydrocarbons, from a syngas, comprising the steps of:

a) preheating a composite catalyst to a temperature lower than a reaction temperature;

b) reducing the composite material;

c) reacting in a fixed bed the gas at the reaction temperature from 150°C to 350°C, at a pressure from $1 \cdot 10^5$ Pa to $5 \cdot 10^6$ Pa and at a gas hour space velocity (GHSV) is in a range from 1000 mLg$^{-1}$h$^{-1}$ to 5000 mLg$^{-1}$h$^{-1}$ in the presence of the composite catalyst;

wherein the composite catalyst comprising:

an alkali-Fe- transition metal oxide wherein the Fe is from 50% to 90% by weight relative to the total weight of the alkali-Fe- transition metal oxide and

a nano molecular sieve zeolite;

and wherein the mass ratio of the metal oxide to the nanosized molecular sieve zeolite is (0.1-10):1.

Preferably the aromatic hydrocarbon obtained in the process is benzene, toluene and/or xylene.

Preferably the metal oxide are irregular particles with a particle size of from 800$\mu$m to 400 $\mu$m, the pore volume of the metal oxide in the catalyst is 0.03-35 cm$^3$/g, and the BET (Brenauer Emmett Teller) specific surface area is 20-300m$^2$/g.

In the present invention the particle size is estimated by transmission electron microscope (TEM) by a Tecnai G220 with an acceleration voltage of 200 KV with a carbon film supported by Cu grid. At least 200 particles were measured to assess the diameter of particles. The volume-surface average diameter was estimated assuming a spherical morphology with the following expression:

$$D_p = \frac{\sum n_i d_i^3}{\sum n_i d_i^2}$$

where ni represents the number of particles with diameter di.

In the present invention, the specific surface area and pore volume were estimated using the Brunauer-Emmett-Teller (BET) and Barrett-Joyner-Halenda (BJH) models, respectively.

The feature of the nano molecular sieve zeolite; has excellent aromatization activity that can effectively inhibit the formation of carbon deposits and significantly improve the value-added aromatics selectivity.

Preferably the pore volume of the nano molecular sieve zeolite is from 0.04 cm$^3$/g to 120 cm$^3$/g, the BET specific surface area is from 50 m$^2$/g to 500 m$^2$/g, and the average pore diameter, measured as a ratio of total pore volume is in a range from 1nm to 10nm.

Preferably the alkali metal is selected from: Na, K, Li, Rb, Cs and Fr. More preferably the molar ratio of alkali metal to iron atoms is (0.03-0.20):1.

Preferably the transition metal is selected from: Ti, Va, Cr, Zr, Ru, Rh, W, Pt, Mn, Co, Ni, Cu and Zn. More preferably Co, Ni, Cu, Mn, Zn or Zr.

Preferably the amount of Fe in the alkali-Fe- transition metal oxide is from 60% to 80% by weight relative to the total weight of the alkali-Fe- transition metal oxide.

Preferably the step a) is carried out in contact with a raw material comprising, the H$_2$ is between 10% to 30% in weight referred to the total weight; CO is between 5% to 15% in weight referred to the total weight; the CO$_2$ is between 1% to 10% in weight referred to the total weight and balanced with N$_2$. Preferably the step a) is carried out at a temperature that is not higher than 300°C, increasing gradually from 150°C to 280°C. Preferably the temperature range is from 150°C to 280°C.

Preferably the syngas comprises CO$_2$ from the 10% to 50 % weight CO$_2$/weight syngas and H$_2$ and CO in a ratio from 1:1 to 1:3.

Preferably the reduction step b) is carried out at a temperature between 350°C to 450°C by reducing with a gas mixture of hydrogen balanced with nitrogen. Particularly the gas mixture of hydrogen balanced with nitrogen is in equal mass flow ratio.

Preferably the temperature of step c) is from 280°C to 340°C, more preferably from 290°C to 340°C; in this way also is sustained high yields of aromatic hydrocarbons over an extended period.

In the present invention the molecular sieve zeolite preferably is a metal modified nano molecular sieves. Preferably this metal is selected from sodium, potassium, iron, niobium, platinum, nickel or molybdenum. More preferably the metal is impregnated in the molecular sieve zeolite and the metal is from 0.1% to 10% of the weight of the molecular sieve zeolite.

Preferably the molecular sieve zeolite is obtained by hydrothermal method. Preferably the molecular sieve zeolite selected from: MCM-22, MCM-49, BETA, or HZSM-5. Particularly HZSM-5.

Preferably the nano molecular sieve zeolite is a metal modified nano molecular sieves. More preferably the nano molecular sieve zeolite is obtained by hydrothermal method.

Preferably the alkali Fe-transition metal oxide and the nano molecular sieve zeolite are pelletized and mixed uniformly.

## Examples

The present invention will be described in more detail with reference to the following examples, but not only limited to these described functions.

## Reaction method

A fixed bed flow type reactor was used for the catalyst evaluation. In the following Examples, unless otherwise stated, high-purity gases containing CO and $H_2$ were used as reaction gases, mixing 10% nitrogen as an internal standard for analysis in an overall feeding rate of 50ml/min, unless otherwise stated.

Described application was in fixed-bed reaction with a double tube fixed bed reactor and a Hastelloy C276 (inner diameter 7 mm), carrying the reaction at the following conditions unless otherwise stated, temperature 300°C, 30 bar pressure with 2000 mL·h$^{-1}$·gcat$^{-1}$ space velocity and syngas volume ratio of $H_2$/CO and CO is 2 for the reaction time of 6-72h.

## Analysis and Evaluation methods

After the reaction gases material is in full contact with the composite catalyst in the reaction tube, an aromatization reaction occurs, and a sufficient amount of $C_2$-$C_4$ olefins and $C_5^+$ hydrocarbons produced on the metal oxide surface are converted into aromatics into the zeolitic cavities, such as benzene, toluene, xylene, trimethylbenzene and heavy aromatics; the reaction product is separated from gas and liquid, and the liquid phase product contains aliphatic $C_5^+$ hydrocarbons, methanol, water and the target product aromatics, the gas phase products contain carrier gas, carbon monoxide, low-carbon alkanes, and low-carbon olefins, etc. The reaction products were analysed separately after passing through the gas-liquid separator. The analysis of liquid phase products was carried out by Clarus 580 gas chromatograph of PerkinElmer Company of the United States, and the analysis of gas phase products was carried out by GC900 gas chromatograph produced by Shanghai Tianpu Instrument. The reaction results are shown in Table 3.

## Example 1. Process for making the catalyst of the invention

This example describes processes for making and characterizing embodiments of the present catalysts.

In particular, this example describes how to make the catalyst of the invention, and also characterizes their ability to process synthesis gas.

An amount of 0.12mol of $Fe(NO_3)_3(H_2O)_9$ with 0.08mol of copper nitrate [$Cu(NO_3)_2$ 3$H_2O$] was dissolved in 190mL deionized water to prepare solution A that was acidified with 1mL of nitric acid followed by heating up to 60°C and stirring for 30 minutes to dissolve completely. Then the precipitant sodium hydroxide was dissolved in deionized water to prepare 1.5M solution B, followed by gradually adding solution B dropwise into solution A using a peristaltic pump until the solution pH=8.0 in a vigorous stirring way at 60°C. After the desired pH point, the aging process of the precipitate was carried out with continuous stirring at the constant temperature of 60°C water for 2 hours. The obtained slurry solution after aging was filtered and washed several times with 300mL water until the final mol ratio of sodium ion to iron atom was adjusted to 0.09 in the precipitate. The obtained solid cake after filtration was dried overnight in an oven for 24 h, usually at a temperature of 120°C, followed by calcination thereof in the air by a muffle furnace for 6 h at 600°C to obtain the sodium-modified iron-copper oxides nanocrystals.

The pore volume of the metal oxide was 0.212cm$^3$/g, the BET specific surface area was 92.9m$^2$/g, and the average pore diameter was 9.1nm.

The zeolite-based oxide solid was synthesized in making a solution A by dissolving a sufficient amount of ethyl orthosilicate, and tetra propylammonium hydroxide in 15mL deionized water at a temperature of 90°C for 48h with another aqueous solution B of aluminum nitrate nonahydrate and sodium hydroxide with the molar ratio of 1:0.3:0.5:0.4:15 for ethyl orthosilicate nonahydrate, aluminium nitrate, tetra propylammonium hydroxide, sodium hydroxide and deionized water, respectively.

The obtained solution B was slowly added dropwise into solution A with vigorous stirring, followed by transferring the obtained initial gel into a hydrothermal autoclave and maintained at 160° C for 48 hours for crystallization followed by centrifugation and several times washing of the product with deionized water until become neutral. The obtained filter cake was then dried at 120°C for 12 hours and calcined at 500°C for 5h to remove the template, and the obtained product was isolated core and washed with deionized water until neutral, the filter cake was dried for 5-18 hours at a temperature of 120°C, with a calcination temperature of 200-600°C for 1-6 hours obtain the Na type nanosized HZSM-5 molecular sieve.

The obtained Na type HZSM-5 was then treated cation exchange process through ammonium form by dispersing it into 1M $NH_4Cl$ solution in vigorous stirring for 5 h, followed by the centrifugation and several (4) times washing with deionized water to remove residual chloride ions. The described ions exchange was completed after the repetition of several (usually 3-4) times, and finally the obtained $NH_4$-HZSM-5 was dried for 24 hours at 120 °C followed by calcining it at 600 °C for 6 h to obtain nanoscale HZSM-5.

The pore volume of nanosized HZSM-5 molecular sieve was $0.340 cm^3/g$, BET specific surface area was $289 m^2/g$, and average aperture was 4.71nm, the ratio of silicon to aluminium is between 50.

The metal oxide prepared in the first step of 2g and the nanosized HZSM-5 molecular sieve prepared in the second step of 2g, respectively were pressed and pelletized into tablets of 20-40 mesh, and uniformly mixed in physical way to obtain the composite catalyst.


**Example 2. Manufacturing value added aromatics from synthesis gas with the catalyst of the invention**

The composite catalyst of above-mentioned 4g was loaded in a fixed-bed tubular reactor for the direct manufacturing of value-added aromatics from synthesis gas. The catalyst bed was heated gradually by purging $N_2$ flow for 2 hours, followed by the catalyst reduction process using hydrogen at 400°C for 6 hours. Then the $H_2$ was replaced by reaction gas mixture of $H_2$/CO and (or) $CO_2$ at the desired reaction conditions of taking the $H_2$/CO gas volume ratio of 2, at the temperature of 350°C, pressure 4MPa, and space velocity $2000.gcat^{-1}.h^{-1}$, for the reaction time of 24h. The raw material gas (CO: $H_2$: $N_2$ = 10:20:20) was pre-heated to 200°C by the preheater and enters the fixed bed reactor.

Unless defined otherwise, the mentioned reaction conditions were kept the same for the overall different catalytic materials, except for exploring the effect of the different parameters such as at different temperatures 280°C, 290°C, 300°C, 310°C, 320°C, 330°C, 340°C and 350°C, pressure 10 bar, 15 bar, 20 bar, 25 bar, 30 bar, 35 bar, 40 bar, space velocity of $1000 mL·h^{-1}·gcat^{-1}$, $1500 mL·h^{-1}·gcat^{-1}$, $2000 mL·h^{-1}·gcat^{-1}$, $2500 mL·h^{-1}·gcat^{-1}$, $3000 mL·h^{-1}·gcat^{-1}$, $3500 mL·h^{-1}·gcat^{-1}$, $4000 mL·h^{-1}·gcat^{-1}$, and syngas ($H_2$/CO) ratio of 1, 2, and 3 (or) with different $CO_2$ composition of 10%, 20%, 30%, 40%, 50%.

The Table 1 includes a chart showing the effect of temperature on CO conversion and $CO_2$ and hydrocarbon selectivity with 3 g of an embodiment of the present catalysts at 30 bar, gas hourly space velocity (GHSV) of $2000 h^{-1}$, and time on stream of 12-48 hours with syngas (20.0% $H_2$, 10.0% CO, 5.0% $CO_2$, and balance $N_2$). The Table 1 shows the variation of CO conversion and $C_1$-$C_5^+$ selectivity at various temperatures and with the other variables remaining constant. The Table 1 also shows that temperature influenced CO conversion that increased from 84% at 290°C. to 97% at 340°C.

Table 1 also shows that the hydrocarbon distribution changed with temperature, where the higher hydrocarbons were relatively more sensitive to temperature, while the light hydrocarbons being favored at higher temperatures.

Table 1. CO conversion and product selectivity obtained over the NaFeCu-HZSM-5 (Si/Al=50) bifunctional catalyst system at varied reaction conditions in the synthesis of aromatic hydrocarbons.

| Reaction conditions | | | | | | Hydrocarbon distribution (C%) | | | | | | Aromatic content in $C_{5+}$ liquid phase [e] C% |
|---|---|---|---|---|---|---|---|---|---|---|---|---|
| T°C | P(bar) | GHSV (mL $g^{-1}h^{-1}$) | $H_2$/CO ratio | CO conv. (%) | $CO_2$ Sel. C% | $CH_4$ | $C_2$-$C_4$ | n-$C_2°$-$C_4°$ | i-$C_4°$ | i-$C_{5+}$[d] | | |
| | | | | | | | | | | i-$C_{5+}$[c] | Aromatics | |
| 280 | 30 | 2000 | 2 | 84.1 | 22.3 | 9.3 | 8.4 | 13.7 | 9.5 | 11.4 | 47.7 | 87.5 |
| 300[a] | 30 | 2000 | 2 | 96.6 | 26.5 | 10.5 | 0.5 | 11.8 | 6.2 | 3.5 | 67.5 | 96.4 |
| 320 | 30 | 2000 | 2 | 97.4 | 29.4 | 18.5 | 0.2 | 16.4 | 6.91 | 3.1 | 54.89 | 98.5 |
| 340 | 30 | 2000 | 2 | 98.8 | 33.1 | 25.4 | 1.4 | 21.2 | 9.2 | 2.4 | 40.4 | 98.7 |

(continued)

| Reaction conditions | | | | | | Hydrocarbon distribution (C%) | | | | | | Aromatic content in $C_{5+}$ liquid phase [e] C% |
|---|---|---|---|---|---|---|---|---|---|---|---|---|
| T°C | P(bar) | GHSV (mL $g^{-1}h^{-1}$) | $H_2$/CO ratio | CO conv. (%) | $CO_2$ Sel. C% | $CH_4$ | $C_2$-$C_4$ | n-$C_2$°-$C_4$° | i-$C_4$° | i-$C_{5+}$[d] | | |
| | | | | | | | | | | i-$C_{5+}$[c] | Aromatics | |
| 300[b] | 30 | 2000 | 2 | 97.1 | 25.8 | 11.1 | 40.4 | 15.2 | 11.3 | 22 | 0 | 0 |

[a] Conducted over Na-FeCu/HZSM-5 catalyst at optimal reaction conditions.
[b] Conducted over FeCu catalyst only.
[c] Confirmed to be mainly olefins
[d] fractions of i-$C_{5+}$ and aromatics in the final collected liquid hydrocarbon phase.
[e] fractions of aromatics in the final collected liquid hydrocarbon phase.

The stability of the composite catalyst prepared in Example 1 of the present invention is significantly better than that of the other molecular sieves, with it has excellent long-range life and commercial application potential.

The Figure 1 shows the effect of time on stream (TOS) on the performance of the catalyst was observed with that of the exemplary best suitable catalyst for 500 h by carrying out the reaction in the same manner as that of Example 1 for optimal test, giving a constant yield of benzene as 75.5 % and 97% CO conversion for a stability test of 50-500 h. The TOS results showed that the activity of the catalyst reached the steady state under the overall reaction period, thus giving a stable catalytic activity with a constant CO conversion over 95% for the overall reaction stream of 50-500 h.

## Example 3. Effect of the pressure

Effect of pressure on CO conversion, $CO_2$ and hydrocarbon selectivity, hydrocarbon distribution, and liquid hydrocarbon distribution was observed at different reaction pressure. The reaction was carried out in the same manner as in Example 2 for the optimal case, except the pressure was changed to different values of 10, 15, 20, 25, 30, and 40 bar, thus giving a maximum yield of 67.5 % aromatics and 97% CO conversion at 30 bar. The higher total pressure could shift the equilibrium towards forward direction that can eventually enhance the CO conversion and change the product distribution as well. The results in Table 2 indicate that the increased pressure has a significant impact on both CO conversion and process selectivity, increasing the CO conversion up to a certain level of 99.1 % at 40 bar from the lower CO conversion level of only 46.5 % at 5 bar. Similarly, the product distribution has also observed an obvious change for decreasing the $CO_2$ and $CH_4$ selectivity from 42 % and 28% at 5 bars to 26 % and 10 % at 30 bars, respectively, while accelerating the overall aromatics contents to 67 % at 30 bars from that lower fraction of only 26 % at 5 bars.

Table 2. CO conversion and product selectivity obtained over the NaFeCu-HZSM-5 (Si/Al =50) bifunctional catalyst system at varied reaction conditions in the synthesis of aromatic hydrocarbons.

| Reaction conditions | | | | | | Hydrocarbon distribution (C%) | | | | | | Aromatic content in $C_{5+}$ liquid phase [e] C% |
|---|---|---|---|---|---|---|---|---|---|---|---|---|
| T°C | P(bar) | GHSV (mL $g^{-1}h^{-1}$) | $H_2$/CO ratio | CO conv. (%) | $CO_2$ Sel. C% | $CH_4$ | $C_2$-C4b | n-$C_2$°-$C_4$ | i-$C_4$° | i-$C_{5+}$[d] | | |
| | | | | | | | | | | i-$C_{5+}$[c] | Aromatics | |
| 300 | 5 | 2000 | 2 | 46.7 | 42.5 | 28.5 | 15.4 | 14.1 | 6.4 | 9.4 | 26.2 | 98.5 |
| 300 | 10 | 2000 | 2 | 55.3 | 37.3 | 23.4 | 13.8 | 15.2 | 6.5 | 10.3 | 30.8 | 97.1 |
| 300 | 15 | 2000 | 2 | 68.2 | 32.6 | 16.9 | 11.1 | 15.9 | 9.4 | 11.5 | 35.2 | 92 |
| 300 | 20 | 2000 | 2 | 82.4 | 29.1 | 16.4 | 8.4 | 14.8 | 8.5 | 9.4 | 42.5 | 85 |
| 300 | 25 | 2000 | 2 | 90.4 | 27.7 | 14.5 | 3.5 | 13.5 | 6.3 | 6.4 | 55.8 | 86.4 |
| 300[a] | 30 | 2000 | 2 | 96.6 | 26.5 | 10.5 | 0.5 | 11.8 | 6.2 | 3.5 | 67.5 | 96.4 |
| 300 | 35 | 2000 | 2 | 98.1 | 25.2 | 13.5 | 0.5 | 10.2 | 8.1 | 8.6 | 59.1 | 70.2 |

(continued)

| Reaction conditions | | | | | | Hydrocarbon distribution (C%) | | | | | | Aromatic content in $C_{5+}$ liquid phase [e] C% |
|---|---|---|---|---|---|---|---|---|---|---|---|---|
| T°C | P(bar) | GHSV (mL $g^{-1}h^{-1}$) | $H_2$/CO ratio | CO conv. (%) | $CO_2$ Sel. C% | $CH_4$ | $C_2$-C4b | n-$C_2$°-$C_4$ | i-$C_4$° | i-$C_{5+}$ [d] | | |
| | | | | | | | | | | i-$C_{5+}$ [c] | Aromatics | |
| 300 | 40 | 2000 | 2 | 99.7 | 29.4 | 11.1 | 0.9 | 13.2 | 5.1 | 18.5 | 51.2 | 51.8 |

[a] Conducted over Na-FeCu/HZSM-5 catalyst at optimal reaction conditions.
[b] Conducted over FeCu catalyst only.
[c] Confirmed to be mainly olefins
[d] fractions of $i$-$C_{5+}$ and aromatics in the final collected liquid hydrocarbon phase.
[e] fractions of aromatics in the final collected liquid hydrocarbon phase.

## Example 4. Effect of different space velocity

The effect of different space velocity on CO conversion, $CO_2$ and hydrocarbon selectivity, hydrocarbon distribution, and liquid hydrocarbon distribution was observed at different gas flow rates. The reaction was carried out in the same manner as in Example 1 for optimal test, except at different space velocity of 1000mL·$h^{-1}$·gcat$^{-1}$, 1500mL·$h^{-1}$gcat$^{-1}$, 2000mL·$h^{-1}$·gcat$^{-1}$, 2500mL·$h^{-1}$·gcat$^{-1}$, 3000mL·$h^{-1}$·gcat$^{-1}$1, 3500mL·$h^{-1}$·gcat$^{-1}$, 4000mL·$h^{-1}$·gcat$^{-1}$. It was observed that enhancing the gas flow rate could increase the CO conversion rate while giving a maximum yield of aromatics as 67.5 % and 97% CO conversion at 2000mL·$h^{-1}$·gcat$^{-1}$. (Table 3). However, an extensive high space time while decreasing the overall contact time of reactant molecules, could also influence the product distribution in decreasing the gasoline range fraction ($C_5$+) while affecting the overall aromatics content in the final product.

Table 3. CO conversion and product selectivity obtained over the NaFeCu-HZSM-5 (Si/Al =50) bifunctional catalyst system at varied reaction conditions in the synthesis of aromatic hydrocarbons.

| Reaction conditions | | | | | | Hydrocarbon distribution (C%) | | | | | | Aromatic content in $C_{5+}$ liquid phase [e] C% |
|---|---|---|---|---|---|---|---|---|---|---|---|---|
| T°C | P(bar) | GHSV (mL $g^{-1}h^{-1}$) | $H_2$/CO ratio | CO conv. (%) | $CO_2$ Sel. C% | $CH_4$ | $C_2$-$C_4$ [b] | n-$C_2$°-$C_4$° | i-$C_4$° | i-$C_{5+}$ [d] | | |
| | | | | | | | | | | i-$C_{5+}$ [c] | Aromatics | |
| 300 | 30 | 1000 | 2 | 85.2 | 38.9 | 19.8 | 12.4 | 21.4 | 8.4 | 14.2 | 23.8 | 84 |
| 300 | 30 | 1500 | 2 | 90.7 | 32.1 | 16.2 | 8.2 | 9.7 | 8.1 | 9.6 | 48.2 | 94.6 |
| 300[a] | 30 | 2000 | 2 | 96.6 | 26.5 | 10.5 | 0.5 | 11.8 | 6.2 | 3.5 | 67.5 | 96.4 |
| 300 | 30 | 2500 | 2 | 91.2 | 27.2 | 11.4 | 0.9 | 11.2 | 10.7 | 9.7 | 56.1 | 95 |
| 300 | 30 | 3000 | 2 | 81.2 | 28.7 | 14.7 | 1.8 | 13.9 | 14 | 11.8 | 43.8 | 98.2 |

[a] Conducted over Na-FeCu/HZSM-5 catalyst at optimal reaction conditions.
[b] Conducted over FeCu catalyst only.
[c] Confirmed to be mainly olefins
[d] fractions of $i$-$C_{5+}$ and aromatics in the final collected liquid hydrocarbon phase.
[e] fractions of aromatics in the final collected liquid hydrocarbon phase.

## Example 5. Effect of different syngas composition

The effect of different syngas composition on CO conversion, $CO_2$ and hydrocarbon selectivity, hydrocarbon distribution, and liquid hydrocarbon distribution was carried at different reaction gas mixture. The reaction was carried out in the same manner as in Example 1 for optimal test, except at different syngas composition with ($H_2$/CO) ratio of 1, 2, and 3 (or) with different $CO_2$ composition of 10%, 20%, 30%, 40%, 50%. It was deduced that the synthesis gas composition can, in some instances, affect both reaction rates and activity giving a maximum yield of aromatics as 67.5 % and 97% CO conversion at contacting a gaseous mixture comprising 20.0% $H_2$, 10% CO, 5% $CO_2$, and balanced $N_2$.

Table 4. CO conversion and product selectivity obtained over the NaFeCu-HZSM-5 (Si/Al =50) bifunctional catalyst system at varied reaction conditions in the synthesis of aromatic hydrocarbons.

| Reaction conditions | | | | | | Hydrocarbon distribution (C%) | | | | | | |
|---|---|---|---|---|---|---|---|---|---|---|---|---|
| T°C | P(bar) | GHSV (mL $g^{-1}h^{-1}$) | $H_2$/CO ratio | CO conv. (%) | $CO_2$ Sel. C% | $CH_4$ | $C_2$-$C_4^b$ | n-$C_2°$-$C_4°$ | i-$C_4°$ | i-$C_{5+}^d$ | | Aromatic content in $C_{5+}$ liquid phase [e] C% |
| | | | | | | | | | | i-$C_{5+}^c$ | Aromatics | |
| 300 | 30 | 2000 | 0.5 | 76.9 | 41.2 | 19.7 | 8.4 | 19.4 | 9.5 | 5.4 | 37.6 | 97.4 |
| 300 | 30 | 2000 | 1 | 87.5 | 36.1 | 15.4 | 5.3 | 16.4 | 6.4 | 3.8 | 52.7 | 98.1 |
| 300[a] | 30 | 2000 | 2 | 96.6 | 26.5 | 10.5 | 0.5 | 11.8 | 6.2 | 3.5 | 67.5 | 96.4 |
| 300 | 30 | 2000 | 3 | 96.7 | 26.5 | 28.7 | 2.4 | 18.2 | 9.4 | 5.2 | 36.1 | 99.3 |

[a] Conducted over Na-FeCu/HZSM-5 catalyst at optimal reaction conditions.
[b] Conducted over FeCu catalyst only.
[c] Confirmed to be mainly olefins
[d] fractions of $i$-$C_{5+}$ and aromatics in the final collected liquid hydrocarbon phase.
[e] fractions of aromatics in the final collected liquid hydrocarbon phase.

## Claims

1. A process to obtain aromatic hydrocarbons, from a syngas, **characterized by** comprising the steps of:

   a) preheating a composite catalyst to a temperature lower than a reaction temperature;
   b) reducing the composite material;
   c) reacting in a fixed bed the gas at the reaction temperature from 150°C to 350°C, at a pressure from $1 \cdot 10^5$ Pa to $5 \cdot 10^6$ Pa and at a gas hour space velocity (GHSV) is in a range from 1000 mLg$^{-1}$h$^{-1}$ to 5000 mLg$^{-1}$h$^{-1}$ in the presence of the composite catalyst;

   wherein the composite catalyst comprising:

   an alkali-Fe- transition metal oxide wherein the Fe is from 50% to 90% by weight relative to the total weight of the alkali-Fe- transition metal oxide and
   a nano molecular sieve zeolite;
   and wherein the mass ratio of the metal oxide to the nanosized molecular sieve zeolite is (0.1-10):1.

2. The process according to the claim 1 **characterized by** the aromatic hydrocarbon obtained in the process is benzene, toluene and/or xylene.

3. The process according to any of the claims 1 to 2 **characterized by** the metal oxide are irregular particles with a particle size of from 800 μm to 400 μm, the pore volume of the metal oxide in the catalyst is from 0.03 cm$^3$/g to 35 cm$^3$/g, and the BET (Brenauer Emmett Teller) specific surface area is from 20 m$^2$/g to 300 m$^2$/g.

4. The process according to any of the claims 1 to 3 **characterized by** the pore volume of the nano molecular sieve zeolite is from 0.04 cm$^3$/g to 120 cm$^3$/g, the BET specific surface area is from 50 m$^2$/g to 500 m$^2$/g, and the average pore diameter, measured as a ratio of total pore volume is in a range from 1nm to 10nm.

5. The process according to any of the claims 1 to 4 **characterized by** the molar ratio of alkali metal to iron atoms is (0.03-0.20):1.

6. The process according to any of the claims 1 to 5 **characterized by** the step a) is carried out the temperature range is from 150°C to 280°C; the reduction step b) is carried out at a temperature between 350°C to 450°C; the temperature of step c) is from 280°C to 340°C,

7. The process according to any of the claims 1 to 6 **characterized by** the syngas comprises $CO_2$ from the 10% to 50 % weight $CO_2$/weight syngas and $H_2$ and CO in a ratio from 1:1 to 1:3.

8. The process according to any of the claims 1 to 7 **characterized by** the nano molecular sieve zeolite is a metal modified nano molecular sieves.

9. The process according to any of the claims 1 to 8 **characterized by** the nano molecular sieve zeolite is obtained by hydrothermal method.

FIG. 1

EP 4 647 418 A1

| | Europäisches Patentamt / European Patent Office / Office européen des brevets | **EUROPEAN SEARCH REPORT** | **Application Number** EP 24 38 2504 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 117 504 923 A (PETROCHINA CO LTD) 6 February 2024 (2024-02-06) * Claims; examples 4-9 * | 1-9 | INV. C07C1/04 C07C15/06 C07C15/04 C07C15/08 |
| A | US 11 014 076 B2 (DALIAN INST CHEM & PHYSICS CAS [CN]) 25 May 2021 (2021-05-25) * the whole document * | 1-9 | B01J23/78 B01J29/40 |

TECHNICAL FIELDS
SEARCHED    (IPC)

C07C
B01J

The present search report has been drawn up for all claims

| Place of search | Date of completion of the search | Examiner |
|---|---|---|
| The Hague | 22 October 2024 | Fritz, Martin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

13

## ANNEX TO THE EUROPEAN SEARCH REPORT
## ON EUROPEAN PATENT APPLICATION NO.

EP 24 38 2504

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2024

| Patent document cited in search report | | Publication date | Patent family member(s) | | Publication date |
|---|---|---|---|---|---|
| CN 117504923 | A | 06-02-2024 | NONE | | |
| US 11014076 | B2 | 25-05-2021 | CN | 107970988 A | 01-05-2018 |
| | | | GB | 2570592 A | 31-07-2019 |
| | | | RU | 2732247 C1 | 14-09-2020 |
| | | | US | 2019262811 A1 | 29-08-2019 |
| | | | WO | 2018076909 A1 | 03-05-2018 |

EPO FORM P0459

For more details about this annex : see Official Journal of the European Patent Office, No. 12/82

**EP 4 647 418 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 10550045 B

- CA 1175073 A